# FASCICULE DE BREVET EUROPEEN

(11) **EP 0 659 720 B1**
(45) Date de publication et mention de la délivrance du brevet: **25.08.1999**
(21) Numéro de dépôt: 94203664.1
(22) Date de dépôt: 16.12.1994
(51) Int. Cl.: C07C 17/42, C07C 19/08

(54) **Compositions stabilisées comprenant du 1,1-dichloro-1-fluoroéthane et utilisation de celles-ci comme agents gonflants dans des prémélanges destinés à la préparation de mousses de polyuréthane**
Stabilisierte Zusammensetzungen die 1,1-Dichlor-1-fluorethan enthalten und ihre Verwendung als Treibmittel in Vormischungen zur Herstellung von Polyurethanschäumen
Stabilised compositions containing 1,1-dichloro-1-fluoroethane and their use as blowing agents in premixtures for the preparation of polyurethane foams

(30) Priorité: 27.12.1993 BE 9301470
(43) Date de publication de la demande: 28.06.1995
(73) Titulaire: SOLVAY (Société Anonyme), 1050 Bruxelles (BE)
(72) Inventeur: Barthelemy, Pierre, B-1315 Pietrebais (BE); Leroy, Annie, B-6637 Fauvillers (BE)
(74) Mandataire: Jacques, Philippe

(56) Documents cités:
- EP-A- 0 539 719
- EP-A- 0 559 264
- CHEMICAL ABSTRACTS, vol. 111, no. 19, 6 Novembre 1989, Columbus, Ohio, US; abstract no. 173592, WATANABE N ET AL 'Stabilization of lower chlorofluoroalkanes containing at least one hydrogen atom' & JP-A-8 950 829 (ASAHI GLASS CO., LTD.;JAPAN ) 27 Février 1989

## Description

La présente invention concerne des compositions stabilisées comprenant du 1,1-dichloro-1-fluoroéthane (HFA-141b).

Les hydrocarbures chlorofluorés entièrement halogénés (CFC) suspectés d'avoir un effet néfaste sur la couche d'ozone peuvent être substitués dans bon nombre d'applications (telles que par exemple l'utilisation comme agent gonflant pour la préparation de mousses, comme solvant, comme fluide caloporteur ou comme gaz propulseur) par des hydrocarbures fluorés comprenant au moins un atome d'hydrogène, encore appelés hydrofluoroalcanes (HFA). Le 1,1-dichloro-1-fluoroéthane (HFA-141b) est un exemple d'hydrofluoroalcane qui s'avère être un substitut intéressant de certains CFC, notamment comme agent gonflant de mousses polymériques et comme solvant.

Il est généralement reconnu que les hydrofluoroalcanes doivent être stabilisés pour éviter tout risque de dégradation en cours de stockage ou d'utilisation. La demande de brevet EP-A-0538085 décrit un procédé de stabilisation du HFA-141b au moyen d'un hydrocarbure éthylénique tel que notamment le styrène, l'alpha-méthylstyrène et l'amylène. La demande de brevet européen EP-A-0559264 (SOLVAY) décrit un procédé de stabilisation d'un hydrofluoroalcane à l'encontre de la dégradation selon lequel on ajoute au dit hydrofluoroalcane de 0,001 à moins de 0,1 % en poids d'un alcool.

Par ailleurs, les HFA peuvent aussi se dégrader dans certaines conditions d'utilisation. Il est par exemple de pratique courante, dans le domaine des mousses de polyuréthane, de préparer des prémélanges de certains composants utilisés ultérieurement pour préparer la mousse. De tels prémélanges comprennent habituellement des quantités appropriées de polyol, d'agent gonflant, de catalyseur, de surfactant et d'agent retardateur de flamme. Les mousses de polyuréthane ou de polyisocyanurate peuvent alors être préparées en faisant réagir un polyisocyanate avec une quantité appropriée d'un tel prémélange. On a toutefois constaté que, dans les prémélanges contenant des polyols, l'hydrofluoroalcane utilisé comme agent gonflant avait tendance à être dégradé en divers produits de dégradation gênants. Ces produits de dégradation peuvent influencer négativement la réactivité du polyol avec le polyisocyanate. Compte tenu du fait que les durées de stockage des prémélanges peuvent être de l'ordre de plusieurs mois, la réactivité des prémélanges contenant les polyols avec le polyisocyanate va décroître au cours du temps, ce qui est, d'un point de vue industrielle, intolérable.

Pour remédier à l'instabilité des hydrofluoroalcanes en présence de composés comprenant des groupements hydroxyle, on propose, dans la demande de brevet japonais JP 01/50829, de les stabiliser à l'aide de dérivés styréniques. Dans la demande de brevet européen EP-A-0539719, on stabilise du HFA-141b au moyen de l'alpha-méthylstyrène et/ou de nitrométhane. Le nitrométhane est préféré. D'autre part, dans la publication Journal of Cellular Plastics, volume 25, 1989, R.M.CROOKER et al., pages 609 à 617, on enseigne que l'addition de l'alpha-méthylstyrène comme stabilisant du HFA-141b n'influence pas sa stabilité dans des prémélanges contenant des polyols.

On a toutefois observé que, même additionné d'alpha-méthylstyrène, le HFA-141b subit encore une dégradation notable au cours du stockage, notamment lorsqu'il est incorporé à des prémélanges contenant des polyols.

L'invention vise à améliorer davantage la stabilité du HFA-141b au stockage, en particulier lorsqu'il est incorporé à un prémélange contenant un polyol, destiné à la fabrication de mousses de polyuréthane.

L'invention concerne dès lors des compositions comprenant du 1,1-dichloro-1-fluoroéthane et un dérivé styrénique répondant à la formule générale 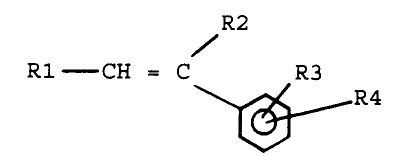 où R1, R2, R3, R4 représentent, indépendamment, un atome d'hydrogène ou un groupement alkyle contenant de 1 à 3 atomes de carbone, caractérisées en ce qu'elles comprennent un co-stabilisant choisi parmi les alcools aliphatiques contenant de 1 à 3 atomes de carbone et moins que 3 fonctions hydroxyle et les alcènes acycliques non fonctionnalisés contenant au moins 4 atomes de carbone.

Les dérivés styréniques selon l'invention répondent à la formule générale 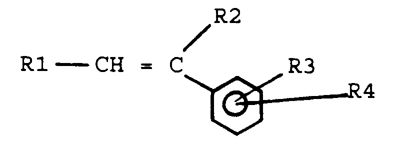 dans laquelle R1, R2, R3, R4 représentent, indépendamment, un atome d'hydrogène ou un groupement alkyle contenant de 1 à 3 atomes de carbone. Des dérivés styréniques préférés sont ceux dans lesquels R1, R3 et R4 représentent un atome d'hydrogène. L'alpha-méthylstyrène est particulièrement préféré.

Des exemples d'alcools utilisables selon l'invention comprennent notamment le méthanol, l'éthanol, le 1-propanol, le 2-propanol et l'éthylèneglycol. L'alcool préféré est le méthanol.

D'une manière avantageuse, les alcènes non fonctionnalisés utilisables dans les compositions selon l'invention ne comportent pas plus que 20 atomes de carbone. Des alcènes acycliques spécialement avantageux sont notamment des alcènes ramifiés. Parmi les alcènes ramifiés le 2-méthyl-2-butène, le 2-méthyl-1-butène, le 3-méthyl-1-butène et leurs mélanges sont préférés. De très bons résultats ont été obtenus avec le 2-méthyl-2-butène industriel, habituellement dénommé amylène, lequel peut contenir un peu de 2-méthyl-1-butène.

Dans les compositions selon l'invention, le co-stabilisant peut être constitué exclusivement d'au moins un alcool ou d'au moins un alcène tels que définis plus haut.

Selon une variante avantageuse de l'invention, le co-stabilisant comprend au moins un alcool et au moins un alcène tels que définis plus haut.

Dans les compositions selon l'invention, la quantité de 1,1-dichloro-1-fluoroéthane est généralement supérieure à 85 % en poids, de préférence supérieure à 95 % en poids. Particulièrement préférées sont les compositions qui contiennent au moins 99 % en poids de 1,1-dichloro-1-fluoroéthane.

Dans les compositions selon l'invention, la quantité du dérivé styrénique est, en général, d'au moins 0,001 % en poids par rapport au poids du 1,1-dichloro-1-fluoroéthane. De préférence, la quantité du dérivé styrénique est d'au moins 0,01 %. En général, la quantité du dérivé styrénique ne dépasse pas 10 % en poids par rapport au poids du 1,1-dichloro-1-fluoroéthane. De préférence, la quantité du dérivé styrénique ne dépasse pas 1 %.

Les compositions selon l'invention, comprennent généralement le co-stabilisant en une quantité au moins égale à 0,0001 % en poids par rapport au poids du 1,1-dichloro-1-fluoroéthane. De préférence, elles comprennent le co-stabilisant en une quantité pondérale au moins égale à 0,001 %. En général, la quantité de co-stabilisant ne dépasse pas 10 % en poids par rapport au poids du 1,1-dichloro-1-fluoroéthane. De préférence, la quantité pondérale en co-stabilisant ne dépasse pas 1 % par rapport au poids du 1,1-dichloro-1-fluoroéthane.

Dans le cas particulier où le co-stabilisant comprend un alcool tel que défini plus haut, l'alcool est avantageusement en une quantité pondérale qui n'excède pas 0,1 % du poids du 1,1-dichloro-1-fluoroéthane.

Les compositions selon l'invention peuvent être constituées essentiellement du 1,1-dichloro-1-fluoroéthane, du dérivé styrénique et du co-stabilisant. En variante, elles peuvent contenir des additifs supplémentaires dont le choix et les teneurs vont dépendre de l'utilisation à laquelle les compositions sont destinées.

Les compositions selon l'invention peuvent être mises en oeuvre comme agent de nettoyage, par exemple comme solvant de dégraissage de pièces métalliques. Elles peuvent également être utilisées comme fluide caloporteur dans les appareils de réfrigération ou comme gaz propulseur pour produits aérosols. Les compositions selon l'invention peuvent avantageusement être mises en oeuvre comme agent gonflant pour la préparation de mousses polymériques, en particulier de mousses de polyuréthane ou de polyisocyanurate.

L'invention concerne dès lors également l'utilisation des compositions selon l'invention comme agent gonflant pour la préparation de mousses polymériques, en particulier de mousses de polyuréthane ou de polyisocyanurate.

L'invention concerne également des prémélanges destinés à la préparation de mousses de polyuréthane ou de polyisocyanurate, comprenant un polyol et un agent gonflant, l'agent gonflant comprenant une composition conforme à l'invention, telle que définie plus haut.

Dans les prémélanges selon l'invention le polyol n'est pas critique et peut être tout polyol communément utilisé pour la fabrication de mousses de polyuréthane ou de polyisocyanurate. Des exemples de polyols utilisables dans les prémélanges selon l'invention sont les polyéthers polyols et les polyesters polyols. La proportion optimum d'agent gonflant par rapport au polyol dans les prémélanges va dépendre de divers paramètres, notamment le type de mousse à préparer et la nature du polyol. Elle peut être déterminée facilement dans chaque cas particulier. En pratique, on utilise généralement, dans les prémélanges selon l'invention, de 1 à 50 parties en poids de la composition selon l'invention pour 100 parties en poids de polyol.

Les prémélanges selon l'invention peuvent n'être constitués que du polyol et de la composition selon l'invention. En variante, ils peuvent contenir un ou plusieurs autres agents gonflants. En règle général, ils comprennent souvent des surfactants, des catalyseurs, des agents retardateurs de flamme et éventuellement d'autres additifs utilisés habituellement pour préparer des mousses de polyuréthane.

Les prémélanges selon l'invention se sont avérés particulièrement stables au stockage. Ils conviennent bien pour la fabrication de mousses de polyuréthane rigides.

L'invention concerne également des mousses de polyuréthane ou de polyisocyanurate obtenues par mise en oeuvre d'une composition conforme à l'invention ou d'un prémélange conforme à l'invention, tels que définis plus haut.

Les exemples qui suivent servent à illustrer l'invention. Parmi ces exemples, les exemples 1R, 2R et 3R sont des exemples de référence, non conformes à l'invention. Les exemples 4, 5 et 6 sont conformes à l'invention.

### Exemple 1R (de référence)

On a préparé un prémélange pour la fabrication d'une mousse de polyuréthane, en mélangeant (quantités pondérales) :
- 70 parties de polyol IXOL®B251 (SOLVAY),
- 130 parties de polyol aminé sur base sucrose CARADOL®585-8 (SHELL),
- 50 parties de trichloropropylphosphate,
- 2 parties de surfactant siliconé TEGOSTAB®B1048 (GOLDSCHMIDT),
- 2 parties d'eau
- 48 parties d'agent gonflant constitué de 1,1-dichloro-1-fluoroéthane (HFA-141b) et 0,1 % en poids d'alpha-méthylstyrène par rapport au poids du HFA-141b,
- 2,4 parties de N,N-diméthyléthanolamine,
- 1,6 partie d'acétate de potassium KACEKAT®KCA (SOLVAY FLUOR UND DERIVATE).

Pour estimer la stabilité du prémélange au stockage, on a procédé à deux mesures successives.

Premièrement, immédiatement après sa préparation, on a mélangé la moitié de ce prémélange avec 147,7 parties de bis-(4-phénylisocyanate) de méthylène MDI®44V20 (BAYER) et on a caractérisé le profil de réactivité de ce prémélange en mesurant 5 paramètres caractéristiques de la mousse de polyuréthane ainsi obtenue : le temps de crème, le temps de fil, le temps de montée, le temps hors colle et le temps de durcissement. Ces temps sont mesurés en prenant comme origine le début de l'agitation du mélange. Ils sont définis de la manière suivante :
- temps de crème : temps à l'issue duquel on observe un changement de coloration du mélange et qui correspond au début de la montée de la mousse;
- temps de fil : temps à partir duquel on commence a observer la formation de fils de polymère lorsqu'une baguette de verre est mise en contact avec la mousse;
- temps de montée : temps mis par la mousse pour atteindre sa hauteur maximale;
- temps hors colle : temps à partir duquel la mousse n'est plus collante;
- temps de durcissement : temps nécessaire pour que la mousse acquière une consistance dure.

L'autre moitié du prémélange a été stockée à 50 °C en présence d'une éprouvette en acier doux. Après 30 jours de stockage à 50 °C, on a mélangé ce prémélange vieilli avec 147,7 parties de bis-(4-phénylisocyanate) de méthylène MDI®44V20 et on a caractérisé le profil de réactivité de ce prémélange en mesurant les 5 paramètres précités.

La stabilité du prémélange est estimée par la variation des paramètres précités au cours du stockage, le prémélange étant d'autant plus stable que cette variation est petite.

Les résultats se trouvent au tableau I.

### Exemple 2R (de référence)

Un prémélange identique au prémélange de l'exemple 1R a été préparé, sauf que l'agent gonflant a consisté en un mélange de 1,1-dichloro-1-fluoroéthane (HFA-141b) et de 0,1 % en poids d'amylène par rapport au poids du HFA-141b.

La stabilité du prémélange a été évaluée comme à l'exemple 1R, en comparant son profil de réactivité après 30 jours de stockage, à 50 °C et en présence d'une éprouvette en acier doux, avec son profil de réactivité initiale. Les résultats se trouvent au tableau I.

### Exemple 3R (de référence)

Un prémélange identique au prémélange de l'exemple 1R a été préparé, sauf que l'agent gonflant a consisté en un mélange de 1,1-dichloro-1-fluoroéthane (HFA-141b) et de 0,01 % en poids de méthanol par rapport au poids du HFA-141b.

La stabilité du prémélange a été évaluée comme à l'exemple 1R, en comparant son profil de réactivité après 30 jours de stockage, à 50 °C et en présence d'une éprouvette en acier doux, avec son profil de réactivité initiale. Les résultats se trouvent au tableau I.

### Exemple 4 (conforme à l'invention)

Un prémélange identique au prémélange de l'exemple 1R a été préparé, sauf que l'on a utilisé pour l'agent gonflant une composition conforme à l'invention, comprenant du 1,1-dichloro-1-fluoroéthane (HFA-141b), 0,05 % en poids d'alpha-méthylstyrène et 0,05 % en poids d'amylène par rapport au poids du HFA-141b.

La stabilité du prémélange a été évaluée comme à l'exemple 1R, en comparant son profil de réactivité après 30 jours de stockage, à 50 °C et en présence d'une éprouvette en acier doux, avec son profil de réactivité initiale. Les résultats se trouvent au tableau I.

### Exemple 5 (conforme à l'invention)

Un prémélange identique au prémélange de l'exemple 1R a été préparé, sauf que l'on a utilisé pour l'agent gonflant une composition conforme à l'invention, comprenant du 1,1-dichloro-1-fluoroéthane (HFA-141b), 0,1 % en poids d'alpha-méthylstyrène et 0,01 % en poids de méthanol par rapport au poids du HFA-141b.

La stabilité du prémélange a été évaluée comme à l'exemple 1R, en comparant son profil de réactivité après 30 jours de stockage, à 50 °C et en présence d'une éprouvette en acier doux, avec son profil de réactivité initiale. Les résultats se trouvent au tableau I.

### Exemple 6 (conforme à l'invention)

Un prémélange identique au prémélange de l'exemple 1R a été préparé, sauf que l'on a utilisé pour l'agent gonflant une composition conforme à l'invention, comprenant du 1,1-dichloro-1-fluoroéthane (HFA-141b), 0,1 % en poids d'alpha-méthylstyrène, 0,005 % en poids d'amylène et 0,01 % en poids de méthanol par rapport au poids du HFA-141b.

La stabilité du prémélange a été évaluée comme à l'exemple 1R, en comparant son profil de réactivité après 30 jours de stockage, à 50 °C et en présence d'une éprouvette en acier doux, avec son profil de réactivité initiale. Les résultats se trouvent au tableau I.

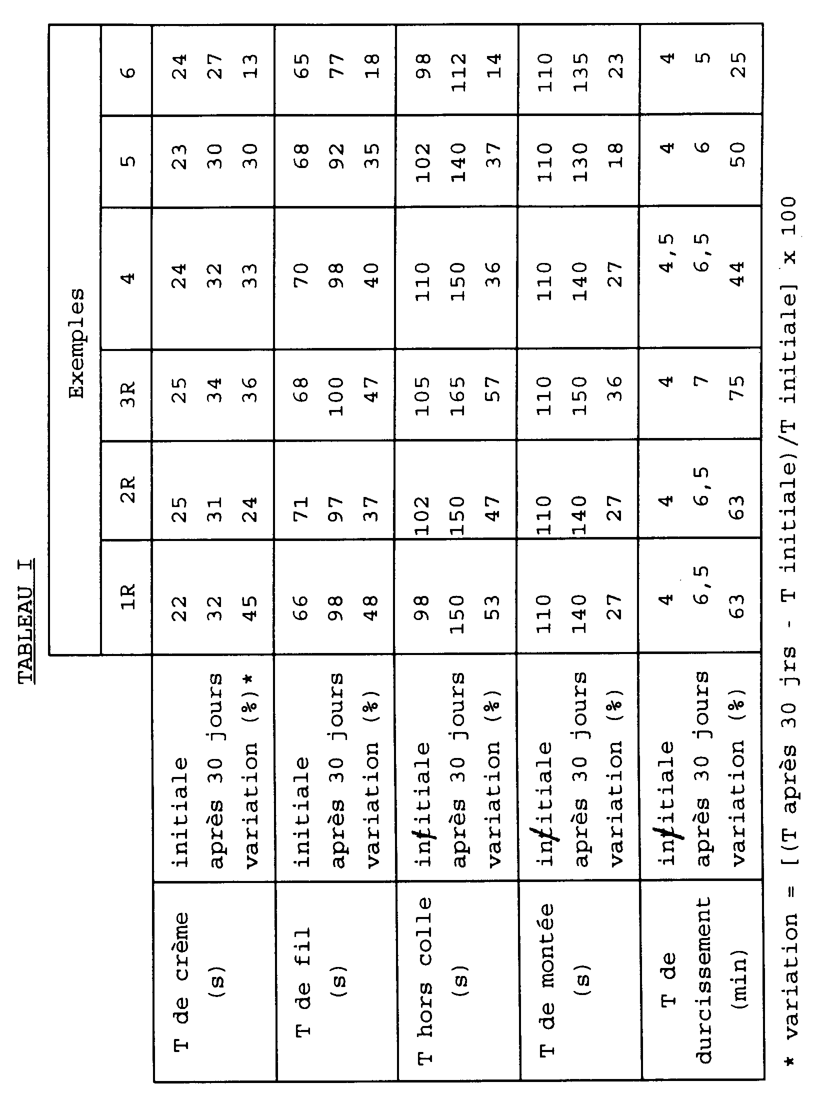

Les résultats dans ce tableau montrent que les prémélanges conformes à l'invention, des exemples 4, 5 et 6, sont plus stables au stockage que les prémélanges des exemples de comparaison 1R, 2R et 3R. Ces résultats sont particulièrement visibles pour ce qui concerne le temps hors colle et le temps de durcissement.

## Revendications

1. Compositions comprenant du 1,1-dichloro-1-fluoroéthane et un dérivé styrénique répondant à la formule générale 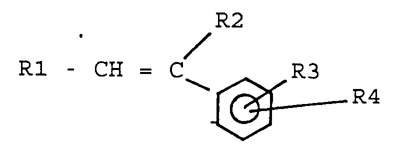 où R1, R2, R3, R4 représentent, indépendamment, un atome d'hydrogène ou un groupement alkyle contenant de 1 à 3 atomes de carbone, caractérisées en ce qu'elles comprennent un co-stabilisant choisi parmi les alcools aliphatiques contenant de 1 à 3 atomes de carbone et moins que 3 fonctions hydroxyle et les alcènes acycliques non fonctionnalisés contenant au moins 4 atomes de carbone.

2. Compositions selon la revendication 1, caractérisées en ce que le co-stabilisant comprend au moins un alcool et au moins un alcène.

3. Compositions selon la revendication 1 ou 2, caractérisées en ce que le dérivé styrénique est sélectionné parmi ceux dans lesquels R1, R3 et R4 représentent un atome d'hydrogène.

4. Compositions selon la revendication 3, caractérisées en ce que le dérivé styrénique est l'alpha-méthylstyrène.

5. Compositions selon l'une quelconque des revendications 1 à 4, caractérisées en ce que la quantité de dérivé styrénique est de 0,01 à 1 % en poids par rapport au poids du 1,1-dichloro-1-fluoroéthane.

6. Compositions selon l'une quelconque des revendications 1 à 5, caractérisées en ce que le co-stabilisant comprend un alcool choisi parmi le méthanol, l'éthanol, le 1-propanol, le 2-propanol et l'éthylèneglycol.

7. Compositions selon la revendication 6, caractérisées en ce que l'alcool est le méthanol.

8. Compositions selon l'une quelconque des revendications 1 à 7, caractérisées en ce que l'alcène est sélectionné parmi le 2-méthyl-2-butène, le 2-méthyl-1-butène, le 3-méthyl-1-butène et leurs mélanges.

9. Compositions selon l'une quelconque des revendications 1 à 8, caractérisées en ce qu'elles comprennent le co-stabilisant en une quantité pondérale de 0,0001 à 10 % par rapport au poids du 1,1-dichloro-1-fluoroéthane.

10. Compositions selon la revendication 9, caractérisées en ce qu'elles comprennent le co-stabilisant en une quantité pondérale de 0,001 à 1 % par rapport au poids du 1,1-dichloro-1-fluoroéthane.

11. Compositions selon la revendication 10, caractérisées en ce que dans le cas où le co-stabilisant comprend un alcool, la quantité pondérale en alcool ne dépasse pas 0,1 % par rapport au poids du 1,1-dichloro-1-fluoroéthane.

12. Utilisation des compositions selon l'une quelconque des revendications 1 à 11 comme agent gonflant pour la préparation de mousses polymériques.

13. Utilisation selon la revendication 12, appliquée aux mousses de polyuréthane ou de polyisocyanurate.

14. Prémélanges destinés à la préparation de mousses de polyuréthane ou de polyisocyanurate comprenant un polyol et un agent gonflant, caractérisés en ce que l'agent gonflant comprend une composition conforme à l'une quelconque des revendications 1 à 11.

15. Mousses de polyuréthane ou de polyisocyanurate obtenues par mise en oeuvre d'un agent gonflant comprenant une composition selon l'une quelconque des revendications 1 à 11.

16. Mousses de polyuréthane ou de polyisocyanurate obtenues par mise en oeuvre d'un prémélange selon la revendication 14.

## Claims

1. Compositions comprising 1,1-dichloro-1-fluoroethane and a styrene derivative corresponding to the general formula 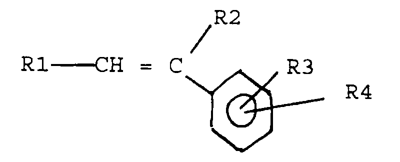 where R1, R2, R3 and R4 independently represent a hydrogen atom or an alkyl group containing from 1 to 3 carbon atoms, characterized in that they comprise a co-stabilizing agent chosen from alcohols containing less than 3 hydroxyl functions and non-functionalized acyclic alkenes containing at least 4 carbon atoms.

2. Compositions according to Claim 1, characterized in that the co-stabilizing agent comprises at least one alcohol at least one alkene.

3. Compositions according to Claim 1 or 2, characterized in that the styrene derivative is selected from those in which R1, R3 and R4 represent a hydrogen atom.

4. Compositions according to Claim 3, characterized in that the styrene derivative is alpha-methylstyrene.

5. Compositions according to any one of Claims 1 to 4, characterized in that the amount of styrene derivative is from 0.01 to 1 % by weight relative to the weight of the 1,1-dichloro-1-fluoroethane.

6. Compositions according to any one of Claims 1 to 5, characterized in that the co-stabilizing agent comprises an aliphatic alcohol selected from methanol, ethanol 1-propanol, 2-propanol and ethylene glycol.

7. Compositions according to Claim 6, characterized in that the alcohol is methanol.

8. Compositions according to any one of Claims 1 to 7, characterized in that the alkene is selected from 2-methyl-2-butene, 2-methyl-1-butene, 3-methyl-1-butene and mixtures thereof.

9. Compositions according to any one of Claims 1 to 8, characterized in that they comprise the co-stabilizing agent in an amount by weight of from 0.0001 to 10% relative to the weight of the 1,1-dichloro-1-fluoro-ethane.

10. Compositions according to Claim 9, characterized in that they comprise the co-stabilizing agent in an amount by weight of from 0.001 to 1% relative to the weight of the 1,1-dichloro-1-fluoroethane.

11. Compositions according to Claim 10, characterized in that in the case where the co-stabilizing agent comprises an alcohol, the amount by weight of alcohol does not exceed 0.1% relative to the weight of the 1,1-dichloro-1-fluoroethane.

12. Use of the compositions according to any one of Claims 1 to 11 as a blowing agent for the preparation of polymer foams.

13. Use according to Claim 12, applied to polyurethane or polyisocyanurate foams.

14. Premixes intended for preparing polyurethane or polyisocyanurate foams comprising a polyol and a blowing agent, characterized in that the blowing agent comprises a composition in accordance with any one of Claims 1 to 11.

15. Polyurethane or polyisocyanurate foams obtained by the use of a blowing agent comprising a composition according to any one of Claims 1 to 11.

16. Polyurethane or polyisocyanurate foams obtained by the use of a premix according to Claim 14.

## Patentansprüche

1. Zusammensetzungen, die 1,1-Dichlor-1-fluorethan und ein Styrolderivat umfassen, das der allgemeinen Formel 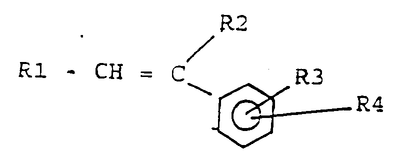 entspricht, worin R₁, R₂, R₃, R₄ unabhängig ein Wasserstoffatom oder eine Alkylgruppe mit 1 bis 3 Kohlenstoffatomen darstellen, dadurch gekennzeichnet, daß sie einen Costabilisator umfassen, der unter den aliphatischen Alkoholen mit 1 bis 3 Kohlenstoffatomen und weniger als 3 Hydroxyfunktionen und den nicht funktionalisierten acyclischen Alkenen mit wenigstens 4 Kohlenstoffatomen ausgewählt ist.

2. Zusammensetzungen gemäß Anspruch 1, dadurch gekennzeichnet, daß der Costabilisator wenigstens einen Alkohol und wenigstens ein Alken umfaßt.

3. Zusammensetzungen gemäß Anspruch 1 oder 2, dadurch gekennzeichnet, daß das Styrolderivat unter denen ausgewählt ist, in denen R₁, R₃ und R₄ ein Wasserstoffatom darstellen.

4. Zusammensetzungen gemäß Anspruch 3, dadurch gekennzeichnet, daß das Styrolderivat alpha-Methylstyrol ist.

5. Zusammensetzungen gemäß einem der Ansprüche 1 bis 4, dadurch gekennzeichnet, daß die Menge an Styrolderivat, bezogen auf das Gewicht des 1,1-Dichlor-1-fluorethans, 0,01 bis 1 Gew.-% beträgt.

6. Zusammensetzungen gemäß einem der Ansprüche 1 bis 5, dadurch gekennzeichnet, daß der Costabilisator einem Alkohol umfaßt, der unter Methanol Ethanol, 1-Propanol, 2-Propanol und Ethylenglykol ausgewählt ist.

7. Zusammensetzungen gemäß Anspruch 6, dadurch gekennzeichnet, daß der Alkohol Methanol ist.

8. Zusammensetzungen gemäß einem der Ansprüche 1 bis 7, dadurch gekennzeichnet, daß das Alken unter 2-Methyl-2-buten, 2-Methyl-1-buten, 3-Methyl-1-buten und deren Gemischen ausgewählt ist.

9. Zusammensetzungen gemäß einem der Ansprüche 1 bis 8, dadurch gekennzeichnet, daß sie den Costabilisator in einer Gewichtsmenge von 0,0001 bis 10%, bezogen auf das Gewicht des 1,1-Dichlor-1-fluorethans, umfassen.

10. Zusammensetzungen gemäß Anspruch 9, dadurch gekennzeichnet, daß sie den Costabilisator in einer Gewichtsmenge von 0,001 bis 1%, bezogen auf das Gewicht des 1,1-Dichlor-1-fluorethans, umfassen.

11. Zusammensetzungen gemäß Anspruch 10, dadurch gekennzeichnet, daß, wenn der Costabilisator einen Alkohol umfaßt, die Gewichtsmenge an Alkohol 0,1%, bezogen auf das Gewicht des 1,1-Dichlor-1-fluorethans, nicht überschreitet.

12. Verwendung der Zusammensetzungen gemäß einem der Ansprüche 1 bis 11 als Treibmittel für die Herstellung von Polymerschäumen.

13. Verwendung gemäß Anspruch 12, angewendet auf Polyurethan- oder Polyisocyanuratschäume.

14. Vormischungen, die zur Herstellung von Polyurethan- oder Polyisocyanuratschäumen bestimmt sind, die ein Polyol und ein Treibmittel umfassen, dadurch gekennzeichnet, daß das Treibmittel eine Zusammensetzung gemäß einem der Ansprüche 1 bis 11 umfaßt.

15. Polyurethan- oder Polyisocyanuratschäume, die durch Verwendung eines Treibmittels, das eine Zusammensetzung gemäß einem der Ansprüche 1 bis 11 umfaßt, erhalten werden.

16. Polyurethan- oder Polyisocyanuratschäume, die durch Verwendung einer Vormischung gemäß Anspruch 14 erhalten werden.
